# EUROPÄISCHE PATENTSCHRIFT

(11) **EP 2 989 453 B1**
(45) Veröffentlichungstag und Bekanntmachung des Hinweises auf die Patenterteilung: **09.09.2020**
(21) Anmeldenummer: 14716566.6
(22) Anmeldetag: 09.04.2014
(51) Int. Cl.: G01N 33/00, G01N 27/22, G01D 11/24

(54) **SENSORBAUELEMENT UND VERFAHREN ZU DESSEN HERSTELLUNG**
SENSOR COMPONENT AND METHOD FOR PRODUCING SAME
COMPOSANT DÉTECTEUR ET PROCÉDÉ POUR LA FABRICATION DUDIT COMPOSANT DÉTECTEUR

(30) Priorität: 22.04.2013 DE 102013104043
(43) Veröffentlichungstag der Anmeldung: 02.03.2016
(73) Patentinhaber: TDK Electronics AG, 81671 München (DE)
(72) Erfinder: OSTRICK, Bernhard, 14513 Teltow (DE); BALZER, Peter, 10179 Berlin (DE)
(74) Vertreter: Epping - Hermann - Fischer
(86) Internationale Anmeldenummer: PCT/EP2014/057171
(87) Internationale Veröffentlichungsnummer: WO 2014/173686

(56) Entgegenhaltungen:
- EP-A1- 2 469 270
- EP-A1- 2 469 270
- WO-A1-02/06808
- WO-A1-2009/015082
- WO-A1-2009/029426
- WO-A2-2004/048955
- DE-A1- 3 911 812
- DE-A1- 10 124 373
- DE-A1- 10 124 373
- DE-A1-102005 000 616
- DE-A1-102007 012 335
- DE-A1-102007 012 335
- REDDY A S G ET AL: "Fully Printed Flexible Humidity Sensor", PROCEDIA ENGINEERING, Bd. 25, 8. Januar 2012 (2012-01-08), Seiten 120-123, XP028436489, ISSN: 1877-7058, DOI: 10.1016/J.PROENG.2011.12.030 [gefunden am 2012-01-08]
- REDDY A S G ET AL: "Fully Printed Flexible Humidity Sensor", PROCEDIA ENGINEERING, vol. 25, 8 January 2012 (2012-01-08), pages 120-123, XP028436489, ISSN: 1877-7058, DOI: 10.1016/J.PROENG.2011.12.030 [retrieved on 2012-01-08]

## Beschreibung

Die vorliegende Erfindung betrifft ein Sensorbauelement und ein Verfahren zur Herstellung des Sensorbauelements.

In DE 10124373 A1 ist ein Temperaturfühler beschrieben, der ein wärmeempfindliches Element aufweist, das in einem Hohlraum eines Gehäuses angeordnet und von dem Gehäuse luftdicht umschlossen wird. Die EP 2469270 A1 betrifft ein Sensorbauelement mit einem Sensorelement zur Erfassung einer Messgröße, das in einem Gehäuse angeordnet ist. Zusätzlich ist innerhalb des Gehäuses ein Bauelement zur drahtlosen Übertragung der Messdaten des Sensorelements vorgesehen.

In Ready et al.: "Fully printed flexible humidity sensor" (Procedia Engineering, Bd. 25) ist ein Feuchtigkeitssensor, der mittels eines kapazitiven Interdigitalwandlers eine Feuchtigkeit messen kann, beschrieben. In DE 10 2005 000 616 A1 ist ein Multifunktions-Sensormodul mit verschiedenen Sensoren zum Erfassen von Umgebungseinflüssen beschrieben.

Eine zu lösende Aufgabe ist es, ein verbessertes Sensorbauelement, insbesondere ein kompaktes Sensorbauelement, sowie ein Verfahren zu dessen Herstellung anzugeben.

Die Aufgabe wird durch das Sensorbauelement mit den Merkmalen des Patentanspruchs 1 sowie durch das Verfahren mit den Merkmalen des Patentanspruchs 13 gelöst. Vorteilhafte Ausgestaltungen und Weiterbildungen sind Gegenstand der abhängigen Patentansprüche.

Ein vorgeschlagenes Sensorbauelement umfasst ein erstes Sensorelement und ein zweites Sensorelement. Das erste Sensorelement und das zweite Sensorelement sind in einem gemeinsamen Gehäuse des Sensorbauelements untergebracht. Weiterhin weist das Sensorbauelement eine Leiterstruktur auf, die eine Elektrodenstruktur und eine von der Elektrodenstruktur gesonderte Anschlussstruktur aufweist. Die Anschlussstruktur ist elektrisch leitfähig mit dem ersten Sensorelement verbunden. Die Elektrodenstruktur ist dem zweiten Sensorelement zugeordnet. Mit Vorteil kann durch das vorgeschlagene Sensorbauelement ein kombiniertes Sensorbauelement angegeben werden, welches beispielsweise gegenüber Sensorbauelementen mit nur einem Sensorelement oder einem Sensor eine Mehrzahl von Messgrößen erfasst sowie gegenüber einer Mehrzahl von einzelnen Sensorbauelementen eine kompaktere Bauform mit geringerem Platzbedarf ermöglicht. Weiterhin ermöglicht das vorgeschlagene Bauelement, den prozesstechnischen Aufwand für die Bestückung des ersten und/oder des zweiten Sensorelements in dem Gehäuse vorteilhaft gering zu halten.

In einer bevorzugten Ausgestaltung ist das Sensorbauelement ein Klimasensorbauelement. Diese Ausgestaltung begünstigt eine Integration des ersten und des zweiten Sensorelements in dem gemeinsamen Gehäuse, da beide Sensorelemente einen vergleichbaren Kontakt oder eine vergleichbare Schnittstelle mit der Umgebung oder dem das Sensorbauelement umgebenden Medium, beispielsweise der Luft, benötigen können. Zum Beispiel kann das erste oder das zweite Sensorelement ein Drucksensor zur Messung des Luftdrucks und das andere Sensorelement ein Feuchtesensor zur Messung der Luftfeuchtigkeit sein. Die Ausgestaltung als Klimasensorbauelement begünstigt weiterhin die kompakte Bauform des Sensorbauelements.

In einer Ausgestaltung ist die Anschlussstruktur von der Elektrodenstruktur beabstandet. Durch diese Ausgestaltung wird mit Vorteil ermöglicht, dass die Anschlussstruktur und die Elektrodenstruktur im Betrieb des Sensorbauelements unterschiedlichen Zwecken dienen.

In einer Ausgestaltung umfasst die Anschlussstruktur einen externen Anschluss. Der externe Anschluss ist elektrisch leitfähig mit der Anschlussstruktur verbunden. Der externe Anschluss stellt vorzugsweise einen externen elektrischen Anschluss des Sensorbauelements zu der Anschlussstruktur dar. Der externe Anschluss kann das Gehäuse des Sensorbauelements durchbrechen, um von außerhalb des Sensorbauelements zugänglich zu sein. Über den externen Anschluss kann mit Vorteil eine Messgröße des ersten Sensorelements von dem Sensorbauelement ausgegeben werden.
In einer Ausgestaltung umfasst die Elektrodenstruktur einen externen Anschluss, welcher elektrisch leitfähig mit der Elektrodenstruktur verbunden ist. Der externe Anschluss der Elektrodenstruktur kann analog zu dem externen Anschuss der Anschlussstruktur ausgebildet sein, um beispielsweise eine Messgröße des zweiten Sensorelements auszugeben.
In einer bevorzugten Ausgestaltung ist das erste Sensorelement ausgebildet, eine erste Messgröße, beispielsweise den Umgebungs- oder Luftdruck oder eine Größe, aus der der Umgebungs- oder Luftdruck ermittelt werden kann, zu erfassen.

Das zweite Sensorelement ist ausgebildet, eine zweite Messgröße, beispielsweise die relative Feuchte oder die Luftfeuchtigkeit oder eine Größe, aus der die relative Feuchte oder die Luftfeuchtigkeit ermittelt werden kann, zu erfassen.
Alternativ kann das zweite Sensorelement so ausgebildet sein, dass die zweite Messgröße eine Gaskonzentration oder den prozentuale Anteil eines Gases, beispielsweise in einer Umgebung oder Gasatmosphäre des Sensorbauelements, darstellt.

Die Elektrodenstruktur ist Teil des zweiten Sensorelements. Gemäß dieser Ausgestaltung kann die zweite Messgröße mit Vorteil über die elektrischen Eigenschaften der Elektrodenstruktur ermittelt werden.

In einer bevorzugten Ausgestaltung ist die Elektrodenstruktur mit einer Beschichtung versehen. Ein Messprinzip des zweiten Sensorelements beruht weiterhin auf einer Wechselwirkung der Elektrodenstruktur mit der Beschichtung. Über die Beschichtung können mit Vorteil die elektrischen Eigenschaften der Elektrodenstruktur, vorzugssweise über die Luftfeuchtigkeit, verändert werden.

In einer Ausgestaltung weist die Elektrodenstruktur ineinandergreifende, elektrisch voneinander getrennte Elektroden auf. Weiterhin ist das Messprinzip des zweiten Sensorelements ein kapazitives Messprinzip. Vorzugsweise sind im Betrieb des Sensorbauelements verschiedene ineinandergreifende Elektroden verschiedenen elektrischen Polaritäten zugeordnet. Die zweite Messgröße wird dabei vorzugsweise über eine Veränderung der elektrischen Kapazität, welche sich beispielsweise nach dem Anlegen einer elektrischen Messspannung zwischen den ineinandergreifenden Elektroden ausbildet, erfasst. Die genannte elektrische Messspannung kann beispielsweise über den externen Anschluss der Elektrodenstruktur an diese angelegt werden.

Vorzugsweise ist oder umfasst die Beschichtung ein Polymer, beispielsweise ein Polyimid, um ein Dielektrikum eines durch die ineinandergreifenden Elektroden gebildeten Kondensators zu bilden. Nach dem Aufbringen der Beschichtung befindet sich diese zweckmäßigerweise zwischen den Elektroden, um das Dielektrikum zu bilden, sowie auch oberhalb der Elektroden.

Die Beschichtung nimmt vorzugsweise abhängig von der relativen Feuchte, Umgebungsfeuchtigkeit oder Luftfeuchtigkeit Feuchtigkeit aus der Luft oder der Umgebung auf oder gibt diese ab, so dass sich die Kapazität des Kondensators verändert. Die Beschichtung kann dabei gemäß einem Potenzgesetz Feuchtigkeit aus der Luft oder der Umgebung aufnehmen oder abgeben. Über eine Kapazitätsänderung kann weiterhin beispielsweise eine Veränderung der Umgebungs- oder Luftfeuchtigkeit von einer elektronischen Einheit ermittelt werden.

In einer Ausgestaltung ist das Messprinzip des zweiten Sensorelements ein resistives Messprinzip. Gemäß dieser Ausgestaltung ist die Elektrodenstruktur zweckmäßigerweise zusammenhängend beziehungsweise nur mit einer Elektrode ausgebildet. Die zweite Messgröße wird dabei vorzugsweise über eine Veränderung des elektrischen Widerstands der Elektrodenstruktur erfasst. Die Veränderung des elektrischen Widerstandes und/oder des elektrischen Leitwertes kann weiterhin beispielsweise über eine Veränderung der Umgebungs- oder Luftfeuchtigkeit von der elektronischen Einheit ermittelt werden.

Die genannte elektronische Einheit kann eine Signalverarbeitungseinheit des Sensorbauelements oder eine externe Einheit sein, welche beispielsweise über den externen Anschluss der Elektrodenstruktur und/oder den externen Anschluss der Anschlussstruktur mit der ersten und/oder der zweiten Messgröße versorgt wird.

In einer bevorzugten Ausgestaltung definiert das Gehäuse einen Innenraum mit Innenflächen, wobei die Anschlussstruktur und die Elektrodenstruktur auf der gleichen Innenfläche oder verschiedenen Innenflächen angeordnet sind. Gemäß dieser Ausgestaltung kann die Anschlussstruktur und die Elektrodenstruktur im Betrieb des Sensorbauelements mit Vorteil gegenüber äußeren Einflüssen geschützt werden. Eine Innenfläche beschreibt eine Fläche auf einer Innenseite des Gehäuses.
In einer bevorzugten Ausgestaltung weist das Sensorbauelement ein erstes Gehäuseteil und ein zweites Gehäuseteil auf, die verbunden sind, um das Gehäuse des Sensorbauelements zu bilden. Mit Vorteil kann durch diese Ausgestaltung eine Bestückung des Gehäuses, beispielsweise mit dem ersten Sensorelement, erleichtert werden. Weiterhin kann auf diese Weise die gesamte Herstellung des Sensorbauelements vereinfacht werden.
Das erste Gehäuseteil kann ein Gehäusekörper sein und das zweite Gehäuseteil kann ein Gehäusedeckel sein, oder umgekehrt.
In einer bevorzugten Ausgestaltung weist das erste und/oder das zweite Gehäuseteil eine Belüftungsöffnung auf. Über die Belüftungsöffnung kann mit Vorteil ein Kontakt des ersten und/oder des zweiten Sensorelements zu der Umgebung, insbesondere der das Sensorbauelement umgebenden Luft oder einer dieses umgebenden Gasatmosphäre, hergestellt werden. Dies ist insbesondere bei einer Ausgestaltung des Sensorbauelements als Klimasensorbauelement zweckmäßig.

In einer bevorzugten Ausgestaltung weist das erste Gehäuseteil und das zweite Gehäuseteil jeweils eine Mehrlagenkeramik auf. Gemäß dieser Ausgestaltung kann das Sensorbauelement mit Vorteil derart mit der Anschlussstruktur und der Elektrodenstruktur versehen werden, dass im Betrieb des Sensorbauelements durch die Anschlussstruktur und/oder durch die Elektrodenstruktur erzeugte Wärme, beispielsweise Joule-Wärme, vorteilhaft abgeleitet werden kann. Weiterhin kann das Sensorbauelement gemäß dieser Ausgestaltung mit Mehrlagenkeramiken besonders kompakt hergestellt sowie beispielsweise gegen äußere elektrische Felder abgeschirmt werden.

In einer bevorzugten Ausgestaltung weist das erste Gehäuseteil eine Hybridkeramik oder Leiterplatte und das zweite Gehäuseteil eine metallische Charakteristik auf, oder umgekehrt. Vorzugsweise besteht das erste oder das zweite Gehäuseteil gemäß dieser Ausgestaltung aus Metall oder umfasst ein Metall. Weiterhin kann das erste oder das zweite Gehäuseteil aus einem Material mit einer metallischen Charakteristik gefertigt sein. Dieses Gehäuseteil kann dabei eine Kappe oder einen Deckel des Sensorbauelements bilden, wohingegen das die Hybridkeramik oder die Leiterplatte aufweisende Gehäuseteil vorzugsweise einen Gehäusekörper darstellt.

In einer Ausgestaltung weist das erste Gehäuseteil und das zweite Gehäuseteil jeweils ein spritzgegossenes Material auf.

Gegenüber der spritzgegossenen Ausführungsform des ersten und des zweiten Gehäuseteil weist die Ausgestaltung des ersten und des zweiten Gehäuseteils als Mehrlagenkeramik den Vorteil einer kompakteren Bauform des Sensorbauelements auf.

In einer Ausgestaltung ist die Anschlussstruktur und/oder die Elektrodenstruktur eben ausgebildet.
In einer Ausgestaltung ist die Leiterstruktur eben ausgebildet.

In einer Ausgestaltung ist die Anschlussstruktur und/oder die Elektrodenstruktur dreidimensional ausgebildet.

Die Ausführungsformen des ersten und des zweiten Gehäuseteils als Mehrlagenkeramik beziehungsweise das spritzgegossenes Material umfassend, ermöglichen mit Vorteil eine Ausführung des ersten und/oder des zweiten Gehäuseteils als dreidimensionalen Schaltungsträger oder als dreidimensionale Schaltung.

In einer bevorzugten Ausgestaltung weist das Sensorbauelement eine Signalverarbeitungseinheit auf, die ausgebildet ist, aus der ersten und/oder der zweiten Messgröße eine abgeleitete Größe, beispielsweise den Wasserdampfpartialdruck oder Feuchtepartialdruck, zu ermitteln. Eine weitere abgeleitete Größe kann die Luftdichte sein. Die Signalverarbeitungseinheit kann alternativ oder zusätzlich ausgebildet sein, bereits die erste und/oder die zweite Messgröße zu ermitteln oder zu deren Ermittlung beizutragen.

Durch die Integration der Signalverarbeitungseinheit in das Sensorbauelement kann die abgeleitete Größe mit Vorteil direkt von dem Sensorbauelement augegeben, und somit auf eine externe elektronische Einheit oder einen von dem Sensorbauelement gesonderten weiteren Signalverarbeitungsbausteins verzichtet werden. Die Signalverarbeitungseinheit kann analog zu der Elektrodenstruktur und zu der Anschlussstruktur einen externen Anschluss aufweisen, so dass eine abgeleitete Größe von dem Sensorbauelements über den genannten externen Anschluss ausgegeben werden kann. Die Signalverarbeitungseinheit ist mit Vorteil elektrisch leitfähig mit der Anschlussstruktur und der Elektrodenstruktur verbunden, so dass bei der Vorsehung der Signalverarbeitungseinheit auf externe Anschlüsse der Elektrodenstruktur und der Anschlussstruktur verzichtet werden kann.

In einer bevorzugten Ausgestaltung weist das Sensorbauelement einen Temperatursensor, beispielsweise zur Messung der Umgebungstemperatur, auf. Der Temperatursensor kann mit der Signalverarbeitungseinheit elektrisch leitfähig verbunden sein. Weiterhin kann eine von dem Temperatursensor gemessene Temperatur, zum Beispiel die Umgebungstemperatur, auf diese Weise an die Signalverarbeitungseinheit übergeben werden. Bevorzugt ist die Signalverarbeitungseinheit geeignet, einen Messwert des Temperatursensors für die Ermittlung einer von dem ersten und/oder dem zweiten Messwert abgeleiteten Größe, zu verwenden.

Der Temperatursensor kann auch als Teil der Signalverarbeitungseinheit ausgeführt sein. Bei dem Temperatursensor kann es sich weiterhin um ein Diode, handeln.

In einer bevorzugten Ausgestaltung ist die Leiterstruktur derart ausgebildet, dass entweder das erste Gehäuseteil oder das zweite Gehäuseteil mit der Elektrodenstruktur und der Anschlussstruktur versehen ist. Gemäß dieser Ausgestaltung weist beispielsweise dasjenige Gehäuseteil, welches nicht mit der Elektrodenstruktur und der Anschlussstruktur versehen ist, genügend Platz für die Signalverarbeitungseinheit auf, so dass eine kompakte Bauform des Sensorbauelements erzielt werden kann.

In einer Ausgestaltung ist die Leiterstruktur derart ausgebildet, dass das erste Gehäuseteil mit der Elektrodenstruktur und das zweite Gehäuseteil mit der Anschlussstruktur versehen ist, oder umgekehrt. Durch diese Ausgestaltung kann mit Vorteil ebenfalls eine kompakte Bauform des Sensorbauelements ermöglicht werden, da die Anschlussstruktur und die Elektrodenstruktur gemäß dieser Ausgestaltung nicht mit ein und demselben Gehäuseteil versehen sind.
Gemäß dieser Ausgestaltung ist das erste Sensorelement vorzugsweise auf dem Gehäuseteil angeordnet, welches mit der Anschlussstruktur versehen ist.
Es wird weiterhin ein Verfahren zur Herstellung des Sensorbauelements angegeben. Das Verfahren umfasst das Bereitstellen des ersten Gehäuseteils und des zweiten Gehäuseteils, wobei das erste Gehäuseteil und das zweite Gehäuseteil ausgebildet sind, ein Gehäuse des Sensorbauelements zu bildenDas Verfahren umfasst weiterhin das Versehen des ersten Gehäuseteils oder beider Gehäuseteile mit der Leiterstruktur. Dabei wird das erste Gehäuseteil oder beide Gehäuseteile über die selben Lithographieschritte oder in einem gemeinsamen Aufdampfprozess mit der Leiterstruktur versehen. Die Leiterstruktur umfasst die Elektrodenstruktur und die elektrisch von der Elektrodenstruktur getrennte Anschlussstruktur. Dabei wird in einem Verfahrensschritt entweder das erste Gehäuseteil mit der Elektrodenstruktur und der Anschlussstruktur versehen oder in einem Verfahrensschritt wird das erste Gehäuseteil mit der Anschlussstruktur und das zweite Gehäuseteil mit der Elektrodenstruktur versehen, um das erste und das zweite Sensorelement elektrisch leitfähig mit der Leiterstruktur zu verbinden. Das Verfahren umfasst weiterhin das Bestücken des Gehäuses mit dem ersten Sensorelement. Das Verfahren umfasst weiterhin das Fertigstellen des Sensorbauelements. Das Fertigstellen des Sensorbauelements kann eine Montage und/oder ein Zusammensetzen des ersten und des zweiten Gehäuseteils umfassen.

In einer Ausgestaltung des Verfahrens wird das erste oder das zweite Gehäuseteil mit der Elektrodenstruktur und der Anschlussstruktur versehen, um das erste und das zweite Sensorelement elektrisch leitfähig mit der Leiterstruktur zu verbinden.

In einer Ausgestaltung des Verfahrens wird das erste Gehäuseteil mit der Anschlussstruktur und das zweite Gehäuseteil mit der Elektrodenstruktur versehen, oder umgekehrt, um das erste und das zweite Sensorelement elektrisch leitfähig mit der Leiterstruktur zu verbinden.

Ein Vorteil des vorgestellten Verfahrens betrifft die Möglichkeit das Gehäuse in ein und demselben Verfahrensschritt mit der Leiterstruktur, umfassend die Elektrodenstruktur und die Anschussstruktur, zu versehen.

Das Sensorbauelement ist vorzugsweise mittels dem hier beschriebenen Verfahren herstellbar oder hergestellt. Insbesondere sind sämtliche für das Verfahren offenbarte Merkmale auch für das Sensorbauelement offenbart und umgekehrt.

In einer bevorzugten Ausgestaltung des Verfahrens wird dasjenige Gehäuseteil, welches mit der Anschlussstruktur versehen ist mit dem ersten Sensorelement bestückt. Durch diese Ausgestaltung kann die elektrisch leitfähige Verbindung zwischen dem ersten Sensorelement und der Anschlussstruktur besonders einfach hergestellt werden.

In einer bevorzugten Ausgestaltung des Verfahrens wird die Elektrodenstruktur mit der Beschichtung versehen, um das zweite Sensorelement zu bilden.

In einer bevorzugten Ausgestaltung weist das Sensorbauelement beziehungsweise das Gehäuse eine Länge zwischen 2,5 mm und 5 mm auf.

In einer bevorzugten Ausgestaltung weist das Sensorbauelement beziehungsweise das Gehäuse eine Breite zwischen 2,5 mm und 5 mm auf.

In einer bevorzugten Ausgestaltung weist das Sensorbauelement beziehungsweise das Gehäuse und eine Höhe zwischen 0,2 mm und 2 mm auf.

Weitere Vorteile, vorteilhafte Ausgestaltungen und Zweckmäßigkeiten der Erfindung ergeben sich aus der folgenden Beschreibung der Ausführungsbeispiele in Verbindung mit den Figuren.
Figur 1A zeigt eine schematische Aufsicht auf eine Innenseite eines ersten Gehäuseteils.
Figur 1B zeigt eine schematische Aufsicht auf ein zweites Gehäuseteil.
Figur 2A zeigt eine schematische Aufsicht auf eine Innenseite des ersten Gehäuseteils entsprechend einer alternativen Ausgestaltung des Sensorbauelements.
Figur 2B zeigt gemäß der in Figur 2A gezeigten Ausgestaltung eine schematische Aufsicht auf eine Innenseite eines zweiten Gehäuseteils des Sensorbauelements.

Gleiche, gleichartige und gleich wirkende Elemente sind in den Figuren mit gleichen Bezugszeichen versehen. Die Figuren und die Größenverhältnisse der in den Figuren dargestellten Elemente untereinander sind nicht als maßstäblich zu betrachten. Vielmehr können einzelne Elemente zur besseren Darstellbarkeit und/oder zum besseren Verständnis übertrieben groß dargestellt sein.

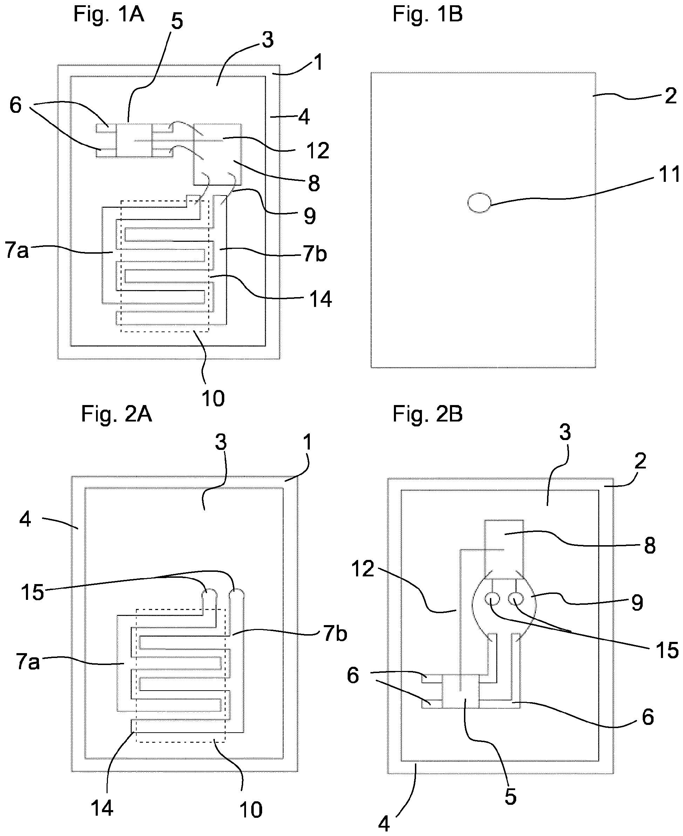

Figur 1A zeigt ein erstes Gehäuseteil 1. Das erste Gehäuseteil 1 ist Teil eines Gehäuses eines Sensorbauelements. Das erste Gehäuseteil 1 weist einen Rand 4 auf. Das erste Gehäuseteil 1 weist weiterhin eine Innenfläche oder Innenseite 3 auf. Das erste Gehäuseteil 1 ist mit einer Anschlussstruktur 6 versehen. Auf der Innenseite 3 des ersten Gehäuseteils 1 ist weiterhin ein erstes Sensorelement 5 angeordnet oder fixiert. Das erste Sensorelement 5 ist elektrisch leitfähig mit der Anschlussstruktur 6 verbunden. Das erste Sensorelement 5 ist ausgebildet, eine erste Messgröße zu erfassen. Vorzugsweise ist diese erste Messgröße der Luftdruck der Umgebung des Sensorbauelements. Das erste Sensorbauelement 5 ist vorzugsweise ein Luftdrucksensor. Die Anschlussstruktur 6 kann einen oder mehrere externe Anschlüsse (nicht explizit dargestellt), beispielsweise zur Ausgabe der ersten Messgröße von dem Sensorbauelement, aufweisen.
Das erste Gehäuseteil 1 ist weiterhin mit einer Elektrodenstruktur 7 versehen. Die Elektrodenstruktur 7 und die Ansdchlussstruktur 6 bilden eine Leiterstruktur (nicht explizit gekennzeichnet), insbesondere eine elektrischer Leiterstruktur, des Sensorbauelements. Die in Figur 1A gezeigte Elektrodenstruktur 7 umfasst ineinandergreifende elektrisch voneinander getrennte Elektroden 7a und 7b. Die beiden ineinandergreifende Elektroden können im Betrieb des Sensorbauelements verschiedenen elektrischen Polaritäten zugeordnet sein und einen Kondensator bilden. Ein elektrisches Feld kann an die Elektroden 7a und 7b beispielsweise über einen oder mehrere externe Anschlüsse (nicht explizit dargestellt) dieser Elektroden angelegt werden.
Die Elektrodenstruktur 7 ist weiterhin zumindest teilweise mit einer Beschichtung 10 versehen oder beschichtet. Die Elektrodenstruktur 7 und die Beschichtung 10 bilden ein zweites Sensorelement 14. Das zweite Sensorelement ist ausgebildet eine zweite Messgröße zu erfassen. Vorzugsweise ist diese zweite Messgröße die relative Feuchte oder Luftfeuchtigkeit der Luft beziehungsweise der das Sensorbauelement umgebenden Gasatmosphäre. Das zweite Sensorbauelement 14 ist vorzugsweise ein Feuchtesensor. Alternativ kann das zweite Sensorbauelement 14 ein Gassensor und die zweite Messgröße beispielsweise eine Gaskonzentration oder der prozentuale Anteil eines Gases in der Luft oder der umgebenden Gasatmosphäre des Sensorbauelements, sein.
Die Beschichtung 10 befindet sich oberhalb und zweckmäßigerweise auch zwischen den Elektroden 7a und 7b, um ein Dielektrikum des durch die ineinandergreifenden Elektroden gebildeten Kondensators zu bilden.

Die Beschichtung 10 kann ein Polymer, beispielsweise ein Polyimid umfassen oder aus diesem bestehen. Die Beschichtung 10 nimmt vorzugsweise abhängig von der relativen Umgebungs- oder Luftfeuchtigkeit des Sensorbauelements Feuchtigkeit auf oder gibt diese ab. Die Beschichtung 10 kann gemäß einem mathematischen Potenzgesetz Feuchtigkeit aus der Luft oder der Umgebung aufnehmen oder abgeben. Die zweite Messgröße kann dabei über eine Veränderung der elektrischen Kapazität der ineinandergreifenden Elektroden erfasst werden, indem sich die Kapazität des durch die Elektroden 7a und 7b gebildeten Kondensators beispielsweise abhängig von der Luftfeuchtigkeit verändert. Diese Kapazitätsänderung kann weiterhin von einer Signalverarbeitungseinheit (siehe unten) des Sensorbauelements erfasst werden.

Auf der Innenseite 3 des ersten Gehäuseteils 1 ist weiterhin eine Signalverarbeitungseinheit 8 angeordnet. Die Signalverarbeitungseinheit 8 ist über eine elektrische Verbindung 12 mit dem ersten Sensorelement 5 verbunden. Über die elektrische Verbindung 12 kann beispielsweise die erste Messgröße des ersten Sensorelements 5 elektrisch an die Signalverarbeitungseinheit 8 übergeben werden. Das erste Sensorelement 5 sowie die Elektroden 7a und 7b können alternativ oder zusätzlich über Bonddrähte 9 (vgl. Figur 1A) mit der Signalverarbeitungseinheit 8 elektrisch leitfähig verbunden sein, beispielsweise um die zweite Messgröße an die Signalverarbeitungseinheit zu übergeben. Die Signalverarbeitungseinheit 8 ist vorzugsweise ausgebildet, aus der ersten und/oder der zweiten Messgröße eine abgeleitete Größe, beispielsweise den Wasserdampfpartialdruck oder Feuchtepartialdruck, zu ermitteln. Eine weitere abgeleitete Größe kann die Luftdichte sein. Für eine Ausgabe der abgeleiteten Größe durch das Sensorbauelement kann die Signalverarbeitungseinheit 8 einen oder mehrere externe (nicht explizit dargestellt) elektrische Anschlüsse aufweisen.

Bevorzugt ist die Signalverarbeitungseinheit 8 geeignet, einen Messwert eines Temperatursensors (nicht explizit dargestellt) des Sensorbauelements für die Ermittlung einer abgeleiteten Größe zu verwenden. Der Temperatursensor kann auch als Teil der Signalverarbeitungseinheit ausgeführt sein. Bei dem Temperatursensor kann es sich um ein Halbleiterbauelement, beispielsweise um eine Diode, handeln. Die Signalverarbeitungseinheit 8 kann alternativ oder zusätzlich ausgebildet sein, bereits die erste und/oder zweite Messgröße zu ermitteln oder zu deren Ermittlung beizutragen.

Das erste Gehäuseteil 1 und das zweite Gehäuseteil 2 können jeweils eine Mehrlagenkeramik umfassen oder daraus bestehen. Weiterhin kann das erste oder das zweite Gehäuseteil 1, 2 eine Hybridkeramik oder eine Leiterplatte aufweisen und das jeweils andere Gehäuseteil 2,1 ein Metall oder eine Metallcharakteristik aufweisen. Weiterhin kann das erste und das zweite Gehäuseteil jeweils ein spritzgegossenes Material umfassen.

In Figur 1B ist ein zweites Gehäuseteil 2 schematisch dargestellt. Das zweite Gehäuseteil 2 und das in Figur 1A gezeigte erste Gehäuseteil 1 bilden vorzugsweise das Gehäuse des beschriebene Sensorbauelement (nicht explizit gekennzeichnet). Nachdem das in Figur 1A gezeigte erste Gehäuseteil 1 einschließlich seiner genannten Komponente mit dem in Figur 1B gezeigten zweiten Gehäuseteil 2 versehen oder die Gehäuseteile zusammengesetzt wurden, ist vorzugsweise das beschriebene Sensorbauelement gebildet. Dieses Sensorbauelement ist vorzugsweise ein Klimasensorbauelement.

Das zweite Gehäuseteil 2 weist eine Belüftungsöffnung 11 auf. Über die Belüftungsöffnung 11 kann ein Kontakt des ersten und/oder zweiten Sensorelements 5, 14 und mit der Umgebung, insbesondere mit der Luft hergestellt werden.

Figur 2A zeigt eine alternative Ausgestaltung eines ersten Gehäuseteils des Sensorbauelements. Das zweite Gehäuseteil 2 ist mit der Elektrodenstruktur 7 versehen. Im Gegensatz zu Figur 1A weist das erste Gehäuseteil 1 lediglich das zweite Sensorelement 14 mit der Elektrodenstruktur 7 und der Beschichtung 10 auf. Das zweite Sensorelement 14 ist ebenfalls auf der Innenseite 3 des ersten Gehäuseteils 1 angeordnet.

Figur 2B zeigt eine schematische Aufsicht auf eine Innenseite 3 des zweiten Gehäuseteils 2. Das zweite Gehäuseteil 2 ist mit der Anschlussstruktur 6 versehen. Die Anschlussstruktur 6, das erste Sensorelement 5 und die Signalverarbeitungseinheit 8 sind in Figur 2B auf einer Innenseite 3 des zweiten Gehäuseteils 2 angeordnet.

Das erste Sensorelement 5 und die Signalverarbeitungseinheit 8 sind weiterhin über die elektrische Verbindung 12 elektrisch leitfähig miteinander verbunden. Weiterhin ist die Anschlussstruktur 6 über die Bonddrähte 9 elektrisch leitfähig mit der Signalverarbeitungseinheit 8 verbunden.

In Figur 2A und in Figur 2B sind beispielhaft jeweils zwei Verbindungspunkte 15 dargestellt. Über die Verbindungspunkte 15 kann nach dem Zusammenbau oder der Montage des ersten Gehäuseteils 1 und des zweiten Gehäuseteils 2 die Elektrodenstruktur 7 vorzugsweise mit der Signalverarbeitungseinheit 8 elektrisch leitfähig verbunden werden, so dass beispielsweise eine zweite Messgröße an die Signalverarbeitungseinheit 8 übergeben werden kann. Die Verbindungspunkte 15 können beispielsweise zum Teil als Leiterbahnen in der Mehrlagenkeramik oder der spritzgegossenen Gehäuseteile 1 und 2 integriert sein. Über die Verbindungspunkte 15 werden das erste Gehäuseteil 1 und das zweite Gehäuseteil 2 für die Montage vorzugsweise verschweißt oder verlötet. Die Verbindungspunkte können anders als in den Figuren dargestellt auch an dem Rand 4 des ersten beziehungsweise des zweiten Gehäuseteils angeordnet sein, um die Montage zu vereinfachen.

Alternativ zu der beschriebenen Elektrodenstruktur 7, kann die Elektrodenstruktur ebenfalls nur eine Elektrode aufweisen, welche zusammen mit einer entsprechenden Beschichtung das zweite Sensorelement 14 bildet. Die Beschichtung kann ebenfalls durch ein Polymer gebildet sein oder ein solches aufweisen. Gemäß dieser Ausgestaltung kann das Messprinzip des zweiten Sensorelements ein resistives Mesprinzip sein, wobei die zweite Messgröße über die Änderung des elektrischen Widerstands und/oder des elektrischen Leitwertes, beispielsweise abhängig von der Luftfeuchtigkeit, erfasst wird.

Der oder die genannten externen Anschlüsse der Anschlussstruktur, der Elektrodenstruktur und/oder der Signalverarbeitungseinheit können jeweils eine Durchkontaktierung von der Innenseite des ersten oder zweiten Gehäuseteils zu einer entsprechenden Außenseite aufweisen. Zur Herstellung des Sensorbauelements werden zunächst das erste und das zweite Gehäuseteil bereitgestellt und diese gemäß der gezeigten Ausgestaltungen oder Ausführungsformen in einem Verfahrensschritt mit der Leiterstruktur versehen. Dabei wird das erste Gehäuseteil oder beide Gehäuseteile über die selben Lithographieschritte oder in einem gemeinsamen Sputter- oder Aufdampfprozess mit der Leiterstruktur versehen. Entsprechend können die Anschlussstruktur und die Elektrodenstruktur zweckmäßigerweise aus dem gleichen Meaterial bestehen. Weiterhin kann das Gehäuse mit dem ersten Sensorelement bestückt werden und das Sensorbauelement zusammengesetzt und fertiggestellt werden.

### Bezugszeichenliste

- 1: Erstes Gehäuseteil
- 2: Zweites Gehäuseteil
- 3: Innenseite
- 4: Rand
- 5: Erstes Sensorelement
- 6: Anschlussstruktur
- 7: Elektrodenstruktur
- 7a, b: Elektrode
- 8: Signalverarbeitungseinheit
- 9: Bonddraht
- 10: Beschichtung
- 11: Belüftungsöffnung
- 12: Elektrische Verbindung
- 14: Zweites Sensorelement
- 15: Verbindungspunkt

## Patentansprüche

1. Sensorbauelement mit einem ersten Sensorelement (5) und einem zweiten Sensorelement (14), wobei das erste Sensorelement (5) ausgebildet ist, eine erste Messgröße, beispielsweise den Umgebungs- oder Luftdruck, zu erfassen und wobei das zweite Sensorelement (14) ausgebildet ist, eine zweite Messgröße, beispielsweise die Luftfeuchtigkeit, zu erfassen, wobei das erste Sensorelement (5) und das zweite Sensorelement (14) in einem gemeinsamen Gehäuse des Sensorbauelements untergebracht sind, wobei das Sensorbauelement eine Leiterstruktur (6, 7) aufweist, die weiterhin eine Elektrodenstruktur (7) und eine gesonderte Anschlussstruktur (6) aufweist, wobei die Anschlussstruktur (6) elektrisch leitfähig mit dem ersten Sensorelement (5) verbunden ist und die Elektrodenstruktur (7) dem zweiten Sensorelement (14) zugeordnet und Teil des zweiten Sensorelements (14) ist, und wobei das Gehäuse einen Innenraum mit Innenflächen (3) definiert und wobei die Anschlussstruktur (6) und die Elektrodenstruktur (7) auf der gleichen Innenfläche (3) oder auf verschiedenen Innenflächen (3) des Gehäuses angeordnet sind, **dadurch gekennzeichnet, dass** die gleiche Innenfläche (3) oder verschiedene Innenflächen (3) des Gehäuses durch die selben Lithographieschritte oder durch einen gemeinsamen Sputter- oder Aufdampfprozess mit der Leiterstruktur für die Anschlussstruktur (6) und die Elektrodenstruktur (7) versehen ist.

2. Sensorbauelement nach Anspruch 1, wobei die Elektrodenstruktur (7) mit einer Beschichtung (10) versehen ist, und wobei ein Messprinzip des zweiten Sensorelements (14) auf einer Wechselwirkung der Elektrodenstruktur (7) mit der Beschichtung (10) beruht.

3. Sensorbauelement nach Anspruch 2, wobei die Elektrodenstruktur (7) ineinandergreifende, elektrisch voneinander getrennte Elektroden (7a, b) aufweist und wobei das Messprinzip des zweiten Sensorelements (14) ein kapazitives Messprinzip ist.

4. Sensorbauelement nach Anspruch 2, wobei das Messprinzip des zweiten Sensorelements (14) ein resistives Messprinzip ist.

5. Sensorbauelement nach einem der Ansprüche 1 bis 4, wobei das Sensorbauelement ein erstes Gehäuseteil (1) und ein zweites Gehäuseteil (2) aufweist, die verbunden sind, um das Gehäuse zu bilden.

6. Sensorbauelement nach Anspruch 5, wobei die Leiterstruktur (6, 7) derart ausgebildet ist, dass entweder das erste Gehäuseteil (1) oder das zweite Gehäuseteil (2) mit der Elektrodenstruktur (7) und der Anschlussstruktur (6) versehen sind.

7. Sensorbauelement nach Anspruch 5 oder 6, wobei die Leiterstruktur (6, 7) derart ausgebildet ist, dass das erste Gehäuseteil (1) mit der Elektrodenstruktur (7) und das zweite Gehäuseteil (2) mit der Anschlussstruktur (6) versehen ist, oder umgekehrt.

8. Sensorbauelement nach einem der Ansprüche 5 bis 7, wobei das erste Gehäuseteil (1) und das zweite Gehäuseteil (2) jeweils eine Mehrlagenkeramik aufweisen.

9. Sensorbauelement nach einem der Ansprüche 1 bis 8, welches eine Signalverarbeitungseinheit (8) aufweist, wobei die Signalverarbeitungseinheit (8) ausgebildet ist, aus der ersten und/oder der zweiten Messgröße eine abgeleitete Größe, wie beispielsweise den Wasserdampfpartialdruck oder den Feuchtepartialdruck, zu ermitteln.

10. Verfahren zur Herstellung eines Sensorbauelements mit folgenden Schritten:
- Bereitstellen eines ersten Gehäuseteils (1) und eines zweiten Gehäuseteils (2), wobei das erste Gehäuseteil (1) und das zweite Gehäuseteil (2) ausgebildet sind, ein Gehäuse des Sensorbauelements zu bilden
- Versehen des ersten Gehäuseteils oder beider Gehäuseteile mit einer Leiterstruktur (6, 7), wobei die Leiterstruktur eine Elektrodenstruktur (7) und eine elektrisch von der Elektrodenstruktur (7) getrennte Anschlussstruktur (6) umfasst, und wobei in einem Verfahrensschritt entweder das erste Gehäuseteil (1) mit der Elektrodenstruktur (7) und der Anschlussstruktur (6) versehen wird, indem eine Innenfläche (3) des ersten Gehäuseteils (1) durch die selben Lithographieschritte oder durch einen gemeinsamen Sputter- oder Aufdampfprozess mit der Leiterstruktur der Anschluss- und Elektrodenstruktur (6, 7)versehen wird, oder in einem Verfahrensschritt das erste Gehäuseteil (1) mit der Anschlussstruktur (6) und das zweite Gehäuseteil (2) mit der Elektrodenstruktur versehen wird, indem durch die selben Lithographieschritte oder durch einen gemeinsamen Sputter- oder Aufdampfprozess die Leiterstruktur der Anschlussstruktur (6) auf eine Innenfläche (3) des ersten Gehäuseteils (1) aufgebracht wird, und die Leiterstruktur der Elektrodenstruktur (7) auf eine Innenfläche (3) des zweiten Gehäuseteils (2) aufgebracht wird, um ein erstes Sensorelement (5) und ein zweites Sensorelement (14) elektrisch leitfähig mit der Leiterstruktur (6, 7) zu verbinden,
- Bestücken des Gehäuses mit dem ersten Sensorelement (5),
- Fertigstellen des Sensorbauelements.

11. Verfahren nach Anspruch 10, wobei dasjenige Gehäuseteil (1, 2), welches mit der Anschlussstruktur (6) versehen ist, mit dem ersten Sensorelement (5) bestückt wird.

12. Verfahren nach Anspruch 10 oder 11, wobei die Elektrodenstruktur (7) mit einer Beschichtung (10) versehen wird, um das zweite Sensorelement (14) zu bilden.

## Claims

1. Sensor component having a first sensor element (5) and a second sensor element (14), wherein the first sensor element (5) is designed to capture a first measurement variable, for example the ambient or air pressure, and the second sensor element (14) is designed to capture a second measurement variable, for example the humidity, wherein the first sensor element (5) and the second sensor element (14) are accommodated in a common housing of the sensor component, wherein the sensor component comprises a conductor structure (6, 7) that furthermore comprises an electrode structure (7) and a separate connection structure (6), wherein the connection structure (6) is connected in an electrically conductive manner to the first sensor element (5) and the electrode structure (7) is allocated to the second sensor element (14) and is part of the second sensor element (14), and wherein the housing defines an interior having internal surfaces (3), and wherein the connection structure (6) and the electrode structure (7) are arranged on the same internal surface (3) or on different internal surfaces (3) of the housing, **characterized in that** the same internal surface (3) or different internal surfaces (3) of the housing is/are provided with the conductor structure for the connection structure (6) and the electrode structure (7) by means of the same lithography steps or by means of a common sputtering or vapour deposition process.

2. Sensor component according to Claim 1, wherein the electrode structure (7) is provided with a coating (10), and wherein a measuring principle of the second sensor element (14) is based on an interaction of the electrode structure (7) with the coating (10).

3. Sensor component according to Claim 2, wherein the electrode structure (7) comprises interengaging electrodes (7a, b) that are electrically separate from one another, and wherein the measuring principle of the second sensor element (14) is a capacitive measuring principle.

4. Sensor component according to Claim 2, wherein the measuring principle of the second sensor element (14) is a resistive measuring principle.

5. Sensor component according to one of Claims 1 to 4, wherein the sensor component comprises a first housing part (1) and a second housing part (2) that are connected in order to form the housing.

6. Sensor component according to Claim 5, wherein the conductor structure (6, 7) is embodied in such a manner that either the first housing part (1) or the second housing part (2) is provided with the electrode structure (7) and the connection structure (6) .

7. Sensor component according to Claim 5 or 6, wherein the conductor structure (6, 7) is embodied in such a manner that the first housing part (1) is provided with the electrode structure (7) and the second housing part (2) is provided with the connection structure (6), or vice versa.

8. Sensor component according to one of Claims 5 to 7, wherein the first housing part (1) and the second housing part (2) comprise in each case a multi-layer ceramic.

9. Sensor component according to one of Claims 1 to 8, which comprises a signal processing unit (8), wherein the signal processing unit (8) is designed to determine a derived variable, for example the water vapour partial pressure or the humidity partial pressure, from the first and/or the second measurement variable.

10. Method for producing a sensor component, having the following steps:
- providing a first housing part (1) and a second housing part (2), wherein the first housing part (1) and the second housing part (2) are designed to form a housing of the sensor component,
- providing the first housing part or the two housing parts with a conductor structure (6, 7), wherein the conductor structure comprises an electrode structure (7) and a connection structure (6) that is electrically separate from the electrode structure (7), and wherein in one method step either the first housing part (1) is provided with the electrode structure (7) and the connection structure (6) by providing an internal surface (3) of the first housing part (1) with the conductor structure of the connection and electrode structures (6, 7) by means of the same lithography steps or by means of a common sputtering or vapour deposition process, or in one method step the first housing part (1) is provided with the connection structure (6) and the second housing part (2) is provided with the electrode structure by applying the conductor structure of the connection structure (6) to an internal surface (3) of the first housing part (1) by means of the same lithography steps or by means of a common sputtering or vapour deposition process and applying the conductor structure of the electrode structure (7) to an internal surface (3) of the second housing part (2), in order to connect a first sensor element (5) and a second sensor element (14) in an electrically conductive manner to the conductor structure (6, 7),
- equipping the housing with the first sensor element (5)
- completing the sensor component.

11. Method according to Claim 10, wherein that housing part (1, 2) which is provided with the connection structure (6) is equipped with the first sensor element (5).

12. Method according to Claim 10 or 11, wherein the electrode structure (7) is provided with a coating (10) in order to form the second sensor element (14).

## Revendications

1. Composant capteur comprenant un premier élément capteur (5) et un deuxième élément capteur (14), le premier élément capteur (5) étant configuré pour acquérir une première grandeur de mesure, par exemple une pression ambiante ou atmosphérique, et le deuxième élément capteur (14) étant configuré pour acquérir une deuxième grandeur de mesure, par exemple l'humidité de l'air, le premier élément capteur (5) et le deuxième élément capteur (14) étant logés dans un boîtier commun du composant capteur, le composant capteur possédant une structure conductrice (6, 7) qui possède en outre une structure d'électrode (7) et une structure de raccordement (6) distincte, la structure de raccordement (6) étant reliée de manière électriquement conductrice au premier élément capteur (5) et la structure d'électrode (7) étant associée au deuxième élément capteur (14) et faisant partie du deuxième élément capteur (14), et le boîtier définissant un espace intérieur ayant des surfaces intérieures (3) et la structure de raccordement (6) et la structure d'électrode (7) étant disposées sur la même surface intérieure (3) ou sur des surfaces intérieures (3) différentes du boîtier, **caractérisé en ce que** la même surface intérieure (3) ou les surfaces intérieures (3) différentes du boîtier sont pourvues de la structure conductrice pour la structure de raccordement (6) et de la structure d'électrode (7) par les mêmes étapes de lithographie ou par un processus de pulvérisation ou de métallisation commun.

2. Composant capteur selon la revendication 1, la structure d'électrode (7) étant pourvue d'un revêtement (10) et le principe de mesure du deuxième élément capteur (14) s'appuyant sur une interaction de la structure d'électrode (7) avec le revêtement (10).

3. Composant capteur selon la revendication 2, la structure d'électrode (7) possédant des électrodes (7a, b) qui s'imbriquent les unes dans les autres, isolées électriquement les unes des autres, et le principe de mesure du deuxième élément capteur (14) étant un principe de mesure capacitif.

4. Composant capteur selon la revendication 2, le principe de mesure du deuxième élément capteur (14) étant un principe de mesure résistif.

5. Composant capteur selon l'une des revendications 1 à 4, le composant capteur possédant une première partie de boîtier (1) et une deuxième partie de boîtier (2), lesquelles sont reliées pour former le boîtier.

6. Composant capteur selon la revendication 5, la structure conductrice (6, 7) étant configurée de telle sorte que soit la première partie de boîtier (1), soit la deuxième partie de boîtier (2) est pourvue de la structure d'électrode (7) et de la structure de raccordement (6).

7. Composant capteur selon la revendication 5 ou 6, la structure conductrice (6, 7) étant configurée de telle sorte que la première partie de boîtier (1) est pourvue de la structure d'électrode (7) et la deuxième partie de boîtier (2) est pourvue de la structure de raccordement (6), ou inversement.

8. Composant capteur selon l'une des revendications 5 à 7, la première partie de boîtier (1) et la deuxième partie de boîtier (2) possédant respectivement une céramique multicouche.

9. Composant capteur selon l'une des revendications 1 à 8, lequel possède une unité de traitement de signal (8), l'unité de traitement de signal (8) étant configurée pour déterminer une grandeur dérivée à partir de la première et/ou de la deuxième grandeur de mesure, comme par exemple la pression partielle de la vapeur d'eau ou la pression partielle de l'humidité.

10. Procédé de fabrication d'un composant capteur comprenant les étapes suivantes :
- fourniture d'une première partie de boîtier (1) et d'une deuxième partie de boîtier (2), la première partie de boîtier (1) et la deuxième partie de boîtier (2) étant configurées pour former un boîtier du composant capteur,
- garniture de la première partie de boîtier ou des deux parties de boîtier d'une structure conductrice (6, 7), la structure conductrice comportant une structure d'électrode (7) et une structure de raccordement (6) isolée électriquement de la structure d'électrode (7), et dans une étape du procédé, soit la première partie de boîtier (1) étant pourvue de la structure d'électrode (7) et de la structure de raccordement (6) en garnissant une surface intérieure (3) de la première partie de boîtier (1) avec la structure conductrice de la structure de raccordement et d'électrode (6, 7) par les mêmes étapes de lithographie ou par un processus de pulvérisation ou de métallisation commun, soit dans une étape du procédé la première partie de boîtier (1) étant pourvue de la structure de raccordement (6) et la deuxième partie de boîtier (2) étant pourvue de la structure d'électrode en appliquant la structure conductrice de la structure de raccordement (6) sur une surface intérieure (3) de la première partie de boîtier (1) et en appliquant la structure conductrice de la structure d'électrode (7) sur une surface intérieure (3) de la deuxième partie de boîtier (2) par les mêmes étapes de lithographie ou par un processus de pulvérisation ou de métallisation commun afin de relier un premier élément capteur (5) et un deuxième élément capteur (14) de manière électriquement conductrice à la structure conductrice (6, 7),
- équipement du boîtier avec le premier élément capteur (5),
- achèvement du composant capteur.

11. Procédé selon la revendication 10, la partie de boîtier (1, 2) qui est pourvue de la structure de raccordement (6) étant équipée du premier élément capteur (5) .

12. Procédé selon la revendication 10 ou 11, la structure d'électrode (7) étant pourvue d'un revêtement (10) en vue de former le deuxième élément capteur (14).
